# EUROPÄISCHE PATENTSCHRIFT

(11) **EP 3 952 832 B1**
(45) Veröffentlichungstag und Bekanntmachung des Hinweises auf die Patenterteilung: **10.09.2025**
(21) Anmeldenummer: 20705319.0
(22) Anmeldetag: 10.02.2020
(51) Int. Cl.: A61K 8/73, A61Q 5/02, A61Q 5/12

(54) **NATÜRLICHES HAARPFLEGESHAMPOO**
NATURAL CONDITIONING SHAMPOO
SHAMPOING NATUREL POUR LE SOIN DES CHEVEUX

(30) Priorität: 08.04.2019 DE 102019204975
(43) Veröffentlichungstag der Anmeldung: 16.02.2022
(73) Patentinhaber: Henkel AG & Co. KGaA, 40589 Düsseldorf (DE)
(72) Erfinder: SCHEELE, Soeren, 25421 Pinneberg (DE); SCHROEDER, Thomas, 22395 Hamburg (DE); METTE, Manuela, 23923 Kleinfeld (DE); WESTPHAL, Petra, 21629 Neu Wulmstorf (DE)
(86) Internationale Anmeldenummer: PCT/EP2020/053320
(87) Internationale Veröffentlichungsnummer: WO 2020/207642

(56) Entgegenhaltungen:
- FR-A1- 2 795 953
- FR-A1- 2 795 954

## Beschreibung

Die Anmeldung beschreibt kosmetische Reinigungsmittel auf der Grundlage von Tensiden, kationischen Inulin-Polymeren und mindestens einem weiteren kationischen Polymeren.

Die Anmeldung beschreibt weiterhin die Verwendung dieser Mittel zur Verbesserung unterschiedlicher Haarpflegeparameter.

Mithilfe kosmetischer Reinigungsmittel, wie Haarshampoos, können menschliche Haare und die Kopfhaut gereinigt und von Talg, Stylingmittelrückständen sowie von anderen Verschmutzungen befreit werden.

Bedingt durch die in kosmetischen Haarreinigungsmitteln üblicherweise enthaltenen (meist anionischen) Tenside geht die Haarreinigung stets auch mit einer Entfernung von Lipiden und Proteinen aus den Haaren oder der Kopfhaut einher, wodurch insbesondere bei häufiger Reinigung eine Schädigung der Haarstruktur und/oder ein Austrocknen der Kopfhaut auftreten kann. Schädigungen der Haarstruktur oder der Haarfasern, insbesondere Spliss und/oder Haarbruch, können zudem durch Umwelteinflüsse (wie beispielsweise intensive Sonneneinstrahlung), mechanische Belastungen (wie beispielsweise Kämmen unter Föhnhitze) sowie durch chemische Einflüsse (wie beispielsweise Färben, Verformen oder Glätten der Haare) begünstigt werden.

Zur Verhinderung und/oder Verminderung von Haarschädigungen wurden Haarshampoos oftmals ölbasierte Pflegestoffe, meist Silikone, hinzugefügt. So beschreibt die französische Patentanmeldung FR 2795953 A1 pflegende Haarshampoos bei denen ein kationisches Fruktan in Verbindung mit einem weiteren Pflegestoff zu Einsatz kommt. Dieser weitere Pflegestoff kann bevorzugt ein Silikon sein.

Die Verwendung von Silikonen in Haareinigungsmitteln ist mittlerweile aber umstritten und weist Nachteile auf, denn Silikone vermindern durch Benetzung der Haaroberfläche die Penetration von Wirk- und Hilfsstoffen in die Haarfasern und erschweren die Frisurengestaltung.

Des Weiteren führt die regelmäßige Anwendung Silikone enthaltender Shampoos mitunter zu einer Überkonditionierung der Haare und einem sogenannten Builtup-Effekt, der durch die Abscheidung und Anhaftung zu großer Silikonmengen auf den Haarfasern zustande kommt.

Dazu kommt, dass Silikone in üblichen Haarreinigungsmitteln meist mithilfe polymerer Verbindungen und/oder alkoxylierter nichtionischer Emulgatoren aufwändig stabilisiert werden müssen, wodurch die Zusammensetzungen unerwünscht komplex werden.

Schließlich ist man aus Gründen der Nachhaltigkeit bestrebt, einen möglichst großen Anteil biologisch abbaubarer Wirkstoffe und/oder Wirkstoffe natürlichen Ursprungs in kosmetischen Produkten einzusetzen.

Die Bereitstellung pflegender kosmetischer Reinigungsmittel wie Haarshampoos, die ,arm an' oder ,frei von' Silikonen, alkoxylierten nichtionischen Emulgatoren und/oder als "Poly..." deklarierten Inhaltsstoffen sind (welche sich nicht aus natürlichen Quellen ableiten), ist daher eine relevante Aufgabe im Bereich der (Haar)Kosmetik.

Silikonarme kosmetische Reinigungsmittel - insbesondere Silikon freie Haarshampoos - weisen im Hinblick auf ihre Haarpflegeeigenschaften oftmals eine schlechtere Produktperformance auf als herkömmliche Formulierungen.

Der vorliegenden Anmeldung lag die Aufgabe zugrunde, kosmetische Reinigungsmittel mit einem höheren Anteil von Wirkstoffen natürlichen Ursprungs zur Verfügung zu stellen, welche die Produktleistung herkömmlicher, Silikone (und/oder als "PEG-..." und/oder als "Poly-..." deklarierter Verbindungen, die nicht aus natürlichen Quellen stammen) enthaltender kosmetischer Reinigungsmittel erreichen.

Durch die Anwendung der kosmetischen Reinigungsmittel sollte neben der schonenden und gründlichen Reinigung von (Kopf)haut und Haaren insbesondere auch eine Verbesserung haarpflegerelevanter Parameter wie beispielsweise Kämmbarkeit, Elastizität, Weichheit gewährleistet werden.

Eine weitere Aufgabe war, durch die Bereitstellung derartiger kosmetischer Reinigungsmittel die Akzeptanz der Verbraucher gegenüber herkömmlichen Produkten zu erhöhen.

Durch die Kombination zweier spezifischer kationischer Polymere bzw. Polymergruppen in Tenside enthaltenden Zusammensetzungen konnten für kosmetische Zwecke geeignete Reinigungsmittel, insbesondere Haarreinigungsmittel, mit hohem Haarpflegepotential zur Verfügung gestellt werden. Diese weisen gegenüber herkömmlichen (auf Silikonen basierenden) Formulierungen mindestens eine vergleichbare bis verbesserte Produktperformance mit dem zusätzlichen Vorteil auf, dass keine weiteren Stabilisierungsmittel eingearbeitet werden müssen, und dass auch bei regelmäßiger Anwendung der Mittel kein Builtup-Effekt auftritt.

Mit den kosmetischen Reinigungsmitteln behandelte Haare lassen sich leicht entwirren und kämmen, sie weisen zudem ein luftig-elastisches Erscheinungsbild, einen weichen Haargriff sowie einen seidigen Glanz auf.

Ein weiterer Vorteil ist, dass sich zur Unterstützung der Pflegeeigenschaften weitere natürliche Pflegewirkstoffe, wie beispielsweise pflanzliche Öle, in die Reinigungsmittel einarbeiten lassen, ohne dass deren Stabilität oder Wirksamkeit negativ beeinträchtigt wird.

Ein erster Gegenstand dieser Anmeldung ist ein kosmetisches Reinigungsmittel, das
a) mindestens ein Tensid, ausgewählt aus anionischen, amphoteren, zwitterionischen und/oder nichtionischen Tensiden,
b) mindestens ein kationisches Inulin-Polymer und
c) mindestens ein weiteres, von b) verschiedenes kationisches Polymer, ausgewählt aus kationischen Polygalactomannanderivaten, dadurch gekennzeichnet, dass das kosmetische Reinigungsmittel (bezogen auf sein Gesamtgewicht) weniger als 0,25 Gew.-% Silikone und weniger als 0,25 Gew.-% alkoxylierte nichtionische Emulgatoren enthält.

Besonders bevorzugte kosmetische Reinigungsmittel enthalten gemäß einer ersten bevorzugten Ausführungsform (bezogen auf das Gewicht des gesamten Mittels)
- 2,50 bis 30,00 Gew.-% mindestens eines Tensids a),
- 0,01 bis 5,00 Gew.-% mindestens eines kationischen Inulin-Polymeren b),
- 0,01 bis 2,00 Gew.-% mindestens eines von von b) verschiedenes kationisches Polymer, ausgewählt aus kationischen Polygalactomannanderivaten.

Die erfindungsgemäßen kosmetischen Reinigungsmittel eignen sich prinzipiell für die Reinigung und/oder die Pflege des gesamten menschlichen Körpers. Besonders geeignet sind sie für die Reinigung und/oder die Pflege stärker behaarter Körperoberflächen und insbesondere für die Reinigung und/oder die Pflege der Kopfhaut und/oder von Kopfhaaren.

Die erfindungsgemäßen kosmetischen Reinigungsmittel enthalten die Wirkstoffe a) bis c) bevorzugt in einem kosmetisch akzeptablen Träger. Darunter wird im Rahmen der Erfindung bevorzugt ein wässriger oder wässrig-alkoholischer Träger verstanden.

Der kosmetische Träger enthält bevorzugt mindestens 75 Gew.-%, mehr bevorzugt mindestens 77 Gew.-%, besonders bevorzugt mindestens 78 Gew.-% und insbesondere bevorzugt mindestens 80 Gew.-% Wasser.

Weiterhin kann der kosmetische Träger 0,01 bis 10 Gew.-%, bevorzugt 0,05 bis 9 Gew.-% und insbesondere 0,10 bis 6 Gew.-% mindestens eines Alkohols enthalten.

Geeignete Alkohole sind beispielsweise Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Die erfindungsgemäßen kosmetischen Reinigungsmittel enthalten als ersten wesentlichen Inhaltsstoff mindestens ein Tensid a), ausgewählt aus anionischen, amphoteren, zwitterionischen und/oder nichtionischen Tensiden.

Die Milde sowie die guten Schaumeigenschaften der erfindungsgemäßen Reinigungsmittel können in besonderem Maße durch die sorgfältige Auswahl der Tensidmengen und/oder der Tensidtypen gesteuert werden. So enthalten die erfindungsgemäßen Reinigungsmittel vorzugsweise mindestens ein anionisches Tensid, welches für die Erzeugung zufriedenstellender Schaummengen und Schaumeigenschaften wesentlich ist. Für die Erzielung einer optimalen Balance zwischen Milde und Schaumeigenschaften der erfindungsgemäßen Mittel hat sich eine Mischung aus mindestens einem anionischen Tensid und mindestens einem milden Co-Tensid herausgestellt, welches vorzugsweise aus amphoteren und/oder zwitterionischen und/oder nichtionischen Tensiden ausgewählt sein kann.

Die Kombination des oder der Tensids(e) a) mit den Komponenten b) und c) wiederum ermöglicht eine Steigerung der relevanten Haarpflegeparameter.

In einer zweiten bevorzugten Ausführungsform enthalten die erfindungsgemäßen kosmetischen Reinigungsmittel daher (bezogen auf das Gewicht des gesamten Mittels)
- 2,50 bis 20,00 Gew.-% mindestens eines anionischen Tensids ai) und/oder
- 0,50 bis 10,00 Gew.-% mindestens eines amphoteren und/oder zwitterionischen Tensids aii),
besonders bevorzugt mindestens ein anionisches Tensid ai) und mindestens ein amphoteres und/oder zwitterionisches Tensid aii) in den zuvor genannten Mengen.

Zu den geeigneten anionischen Tensidtypen ai), die in den erfindungsgemäßen Mitteln eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe (Sarcosinat-Tenside),
- Acyltauride mit 8 bis 24 C-Atomen in der Acylgruppe (Taurat-Tenside),
- Acylisethionate mit 8 bis 24 C-Atomen in der Acylgruppe (Isethionat-Tenside),
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen (Sulfosuccinat-Tenside),
- Alpha-Olefinsulfonate mit 8 bis 24 C-Atomen (alpha-Olefinsulfonat-Tenside),
- Alkylsulfate und/oder Alkylethersulfatsalze der Formel R-(OCH₂-CH₂)ₙ-O-SO₃X, in der R bevorzugt eine geradkettige oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 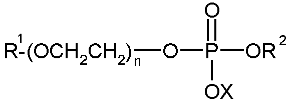
in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Besonders bevorzugt sind Alkylsulfat- und/oder Alkylethersulfatsalze, (Salze von) Ethercarbonsäuren, Sarcosinate, Isethionate, Taurate, Sulfosuccinate und/oder Alpha-Olefinsulfonate, insbesondere Alkylsulfat- und/oder Alkylethersulfatsalze.

Das oder die anionische Tensid(e) ai) wird (werden) in den erfindungsgemäßen Reinigungsmitteln (bezogen auf das Gewicht des gesamten Mittels) bevorzugt in einer Menge von 2,50 bis 20,00 Gew.-%, mehr bevorzugt von 3,00 bis 18,00 Gew.-%, besonders bevorzugt von 4,00 bis 16,00 Gew.-%, ganz besonders bevorzugt von 5,00 bis 14,00 Gew.-% und insbesondere von 6,00 bis 12,50 Gew.-% eingesetzt.

In einer ganz bevorzugten Ausführungsform wird als anionisches Tensid ai) mindestens ein(e) Alkylsulfat- und/oder Alkylethersulfatsalz, Ethercarbonsäure(salz), Sarcosinat, Isethionat, Taurat, Sulfosuccinat und/oder Alpha-Olefinsulfonat, insbesondere ein Alkylsulfat- und/oder Alkylethersulfatsalz, in den zuvor genannten Mengen in den erfindungsgemäßen Reinigungsmitteln eingesetzt.

Zu den geeigneten amphoteren und/oder zwitterionischen Tensidtypen aii), die in den erfindungsgemäßen kosmetischen Reinigungsmitteln eingesetzt werden können, zählen beispielsweise eine oder mehrere Verbindungen der nachfolgenden Formeln (i) bis (vii). Darin steht der Rest R vorzugsweise für einen geradkettigen oder verzweigten, gesättigten oder ein- oder mehrfach ungesättigten Alkyl- oder Alkenylrest mit 7 bis 23 Kohlenstoffatomen (Formeln (i) und (ii)) oder für einen geradkettigen oder verzweigten, gesättigten oder ein- oder mehrfach ungesättigten Alkyl- oder Alkenylrest mit 8 bis 24 Kohlenstoffatomen (Formeln (iii) bis (vii)):

| | |
|---|---|
| 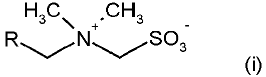 | 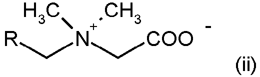 |
| 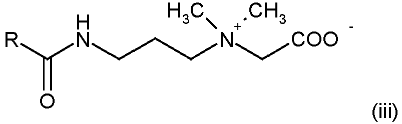 | 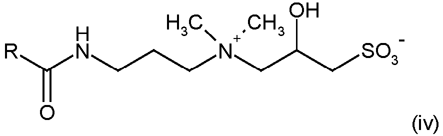 |
| 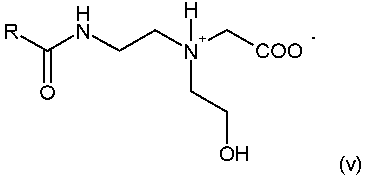 | 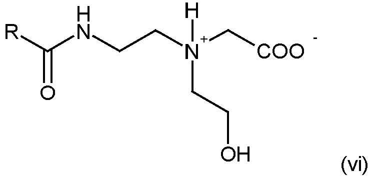 |
| 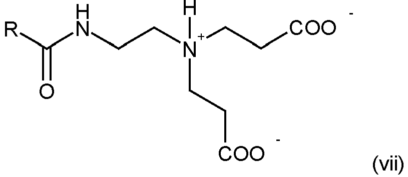 | |

Bevorzugte amphotere Tenside der zuvor genannten Formeln (i) bis (vii) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 8 bis 16 C-Atomen. Besonders bevorzugt sind amphotere Tenside b), bei denen sich der Rest R von Kokosfett ableitet. Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren Tenside.

Insbesondere bevorzugt sind Tenside mit den INCI-Bezeichnungen Cocamidopropylbetain, Lauramidopropylbetain, Cocoampho(di)acetate und/oder Lauroapho(di)acetate.

Das oder die amphotere(n) und/oder zwitterionische(n) Co-Tensid(e) aii) wird (werden) in den erfindungsgemäßen kosmetischen Reinigungsmitteln (bezogen auf deren Gesamtgewicht) vorzugsweise in einer Menge von 0,50 bis 10,00 Gew.-%, mehr bevorzugt von 0,75 bis 8,00 Gew.-%, besonders bevorzugt von 1,00 bis 6,00 Gew.-% und insbesondere von 1,25 bis 5,00 Gew.-% eingesetzt.

In einer weiteren besonders bevorzugten Ausführungsform enthalten die erfindungsgemäßen kosmetischen Reinigungsmittel
- als anionisches Tensid ai) mindestens eine Verbindung aus der Gruppe der Alkyl(ether)sulfate, Sarcosinate, Taurate, Isethionate, Sulfosuccinate und/oder Olefinsulfonate, und
- als amphoteres und/oder zwitterionisches Tensid aii) mindestens eine Verbindung aus der Gruppeder Alkylampho(di)acetate und/oder -propionate und/oder Alkylamidopropylbetaine.

Besonders bevorzugte anionische Tenside im Sinne der vorliegenden Erfindung sind aufgrund ihrer hervorragenden Schaumeigenschaften Alkyl(ether)sulfate ai). Diese werden - zur Steigerung der Milde - vorzugsweise mit Cocamidopropylbetain und/oder Cocoampho(di)acetate als Co-Tensid aii) kombiniert.

Für einige Anwendungsformen - beispielsweise für die Reinigung und Pflege stark vorgeschädigter Haare und/oder besonders feiner Haare und/oder von Baby- oder Kleinkinderhaaren - kann es von Vorteil sein, auf die Verwendung von Sulfattensiden zu verzichten.

Ein Verzicht auf Sulfattenside geht oftmals mit einer dramatischen Verschlechterung der Schaumeigenschaften (Quantität und Qualität) einher, so dass die Auswahl geeigneter Tenside sehr mühsam ist. Die Einarbeitung ölbasierter Pflegestoffe in Haarreinigungsmittel wirkt sich zudem negativ auf die Stabilität, die Viskosität und die Schaumeigenschaften der Mittel aus.

Es wurde gefunden, dass pflegende Haarreinigungsmittel mit guten Schaumeigenschaften und hervorragendem Pflegepotential hergestellt werden können, wenn als anionische, sulfatfreie Tensidbasis mindestens ein Tensid aus der Gruppe der anionischen Taurat-Tenside und/oder Isethionat-Tenside und/oder alpha-Olefinsulfonat-Tenside gewählt wird. Vorzugsweise wird mindestens eines dieser Tenside mit Cocamidopropylbetain und/oder Cocoampho(di)acetate als Co-Tensid kombiniert.

Geeignete nichtionische Tenside für die Verwendung als Co-Tensid sind beispielsweise
- Aminoxide, die ausgewählt sein können aus Verbindungen der allgemeinen Formeln (I) oder (II) 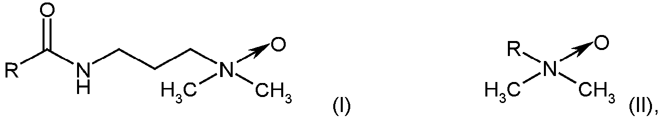
   in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.
   Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (I) oder (II).
- Fettsäurealkanolamide der nachfolgenden allgemeinen Formel, 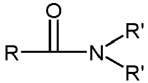 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Gemische aus Alkyl-(oligo)-glucosiden und Fettalkoholen zum Beispiel das im Handel erhältliche Produkt Montanov^{®}68,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden,
- Alkyl(oligo)glycoside. Geeignete Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.
   Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann. Besonders bevorzugte Alkyl(oligo)glycoside weisen einen Oligomerisierungsgrad zwischen 1,2 und 1,5 auf. Der Rest R steht bevorzugt für mindestens einen Alkyl- und/oder Alkenylrest mit 4 bis 24 C-Atomen.
   Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Besonders bevorzugte nichtionische Tenside, die in den erfindungsgemäßen Mitteln als Co-Tensid enthalten sein können, sind Fettsäurealkanolamide, Alkyl(oligo)glucoside und Aminoxide. Insbesondere bevorzugt sind Fettsäurealkanolamide.

Das oder die nichtionische(n) Co-Tensid(e) wird (werden) in den erfindungsgemäßen kosmetischen Reinigungsmitteln (bezogen auf deren Gesamtgewicht) vorzugsweise in einer Menge von 0,05 bis 3,00 Gew.-%, mehr bevorzugt von 0,10 bis 2,50 Gew.-%, besonders bevorzugt von 0,20 bis 2,25 Gew.-% und insbesondere von 0,30 bis 2,00 Gew.-% eingesetzt.

Insbesondere bevorzugt wird in den erfindungsgemäßen Mitteln ein Fettsäurealkanolamid in den zuvor genannten Mengen eingesetzt.

Eine zweite wesentliche Komponente in den erfindungsgemäßen kosmetischen Reinigungsmitteln ist ein kationisches Inulin-Polymer b).

Ein Gehalt dieser spezifischen Polymertypen in der erfindungsgemäßen Wirkstoffmischung ist nicht nur für die Verbesserung der Haarpflegeeigenschaften von Vorteil, sondern es wurde zudem gefunden, dass Polymere b) in Kombination mit den weiteren kationischen Polymeren c) auch nach regelmäßiger Anwendung keinen Überkonditionierungseffekt verursachen.

Inulin ist ein Polysaccharid, das zu der Gruppe der Fructane gehört. Es umfasst in der Kette neben einem endständigen Glucosebaustein bis zu 60 Fructosemonomere, die jeweils über β-2,1-glykosidische Bindungen miteinander verknüpft sind.

Inulin kann aus den Blättern, Wurzeln, Früchten und/oder Blüten von Korb- und/oder Doldenblütlern, wie beispielsweise Topinambur, Chicorée, Artischocke und/oder Pastinake, gewonnen werden.

Erfindungsgemäß besonders geeignete kationische Inulin-Polymere b) werden kationisch modifiziert, indem Hydroxylgruppen der Fructosebausteine mit reaktiven quaternären Ammoniumverbindungen zur Reaktion gebracht werden. Als geeignete quaternäre Ammoniumverbindungen kommen vorzugsweise Verbindungen der nachfolgenden Formel

N+(R¹R²R³R⁴) X⁻

in Frage, in der
R¹, R² und R³ für Methyl- oder Ethylgruppen und
R⁴ für eine Epoxy-R⁵- oder eine Halohydringruppe Y-CH₂-CH(OH)-R⁵- steht, in der R⁵ eine C₁-C₃-Alkylengruppe, Y ein Halogenid und X ein Anion wie Cl⁻, Br, I⁻ oder HSO4⁻ steht.
Ganz besonders geeignete kationische Inulin-Polymere b) im Sinne der vorliegenden Erfindung entsprechen der Formel

   R-O-CH₂-CH(OH)-R⁵-N⁺(R¹R²R³) X⁻,
worin R Inulin ist und die anderen Reste dieselbe Bedeutung wie oben haben.

In einer dritten besonders bevorzugten Ausführungsform enthalten die erfindungsgemäßen Haarreinigungsmittel kationische Inulin-Polymere b), die mit kationischen Hydroxy-C₁-C₃-alkyl-trialkylammonium-Gruppen, insbesondere mit Hydroxypropyltrimethylammoniumgruppen, kationisch modifiziert wurden.

Innerhalb dieser Ausführungsform sind insbesondere unter der INCI-Bezeichnung Hydroxypropyltrimonium Inulin bekannte und im Handel erhältliche kationische Inulin-Polymere b) bevorzugt.

Der kationische Substitutionsgrad der kationischen Inulin-Polymere b), insbesondere von unter der INCI-Bezeichnung Hydroxypropyltrimonium Inulin bekannten kationischen Inulinen, kann variiert und je nach Bedarf eingestellt werden.

Für die Verwendung in den erfindungsgemäßen kosmetischen Reinigungsmitteln hat es sich als besonders bevorzugt erwiesen, wenn derart kationisch modifizierte Inulin-Polymere b) einen höheren Grad der kationischen Modifizierung (höherer kationischer Substitutionsgrad) aufweisen, denn dadurch kann in den Mitteln eine bessere Coacervatbildung und letztendlich eine bessere Pflegeleistung erzielt werden.

In einer vierten besonders bevorzugten Ausführungsform weist das in den erfindungsgemäßen kosmetischen Reinigungsmitteln enthaltene kationische Inulin-Polymer b) eine kationische Ladungsdichte > 1,5 meq/g, mehr bevorzugt > 2,0 meq/g, besonders bevorzugt > 2,5 meq/g, ganz besonders bevorzugt > 3,0 meq/g und insbesondere > 3,5 meq/g auf.

Innerhalb dieser Ausführungsform ist es insbesondere bevorzugt, wenn unter der INCI-Bezeichnung Hydroxypropyltrimonium Inulin bekannte kationische Inulin-Polymere b) eine kationische Ladungsdichte > 1,5 meq/g, mehr bevorzugt > 2,0 meq/g, besonders bevorzugt > 2,5 meq/g, ganz besonders bevorzugt > 3,0 meq/g und insbesondere > 3,5 meq/g aufweisen.

In einer fünften besonders bevorzugten Ausführungsform weist das in den erfindungsgemäßen kosmetischen Reinigungsmitteln enthaltene kationische Inulin-Polymer b) eine mittlere Molmasse von 2,000 bis 50,000 g/mol, mehr bevorzugt 2,500 bis 40,000 g/mol, besonders bevorzugt 3,000 bis 30,000 g/mol, ganz besonders bevorzugt 3,500 bis 20,000 g/mol und insbesondere 4,000 bis 10,000 g/mol auf.

Innerhalb dieser Ausführungsform ist es insbesondere bevorzugt, wenn unter der INCI-Bezeichnung Hydroxypropyltrimonium Inulin bekannte kationische Inulin-Polymere b) eine mittlere Molmasse von 2,000 bis 50,000 g/mol, mehr bevorzugt 2,500 bis 40,000 g/mol, besonders bevorzugt 3,000 bis 30,000 g/mol, ganz besonders bevorzugt 3,500 bis 20,000 g/mol und insbesondere 4,000 bis 10,000 g/mol aufweisen.

Das oder die kationische(n) Inulin-Polymer(e) b) - vorzugsweise unter der INCI-Bezeichnung Hydroxypropyltrimonium Inulin bekannte Verbindungen b) - werden in den erfindungsgemäßen kosmetischen Reinigungszusammensetzungen bevorzugt in einer Menge von 0,01 bis 5,00 Gew.-%, mehr bevorzugt 0,02 bis 4,00 Gew.-%, besonders bevorzugt 0,03 bis 3,00 Gew.-%, ganz besonders bevorzugt 0,04 bis 2,50 Gew.-% und insbesondere von 0,05 bis 2,00 Gew.-% eingesetzt (bezogen auf das Gesamtgewicht der Reinigungszusammensetzungen).

Eine dritte wesentliche Komponente in den erfindungsgemäßen kosmetischen Reinigungsmitteln ist ein weiteres, von b) verschiedenes kationisches Polymer c).

Es wurde gefunden, dass die Kombination kationischer Inulin-Polymere b) und spezifischen, aus natürlichen Quellen erhältlichen kationischen Polymere c) besonders geeignet für die Erzielung sehr guter Haarpflegeeffekte ist.

Erfindungsgemäß handelt es sich bei den aus natürlichen Quellen stammenden kationischen Polymere c) um kationische Polygalactomannanderivate.

In einer weiteren bevorzugten Ausführungsform enthalten die erfindungsgemäßen kosmetischen Reinigungsmittel mindestens ein von b) verschiedenes kationisches Polymer c), das aus kationischen Polymeren natürlichen Ursprungs ausgewählt ist, nämlich aus kationischen Polygalactomannanderivaten.

Galactomannane sind Polysaccharide, die aus Kombinationen von Mannose- und Galactose-Monomeren in unterschiedlichen Gehalten bestehen. Darin sind die Mannoseeinheiten untereinander über ß(1-4)-glykosidische Bindungen verbunden; die Galactoseeinheiten über α(1-6)-Bindungen. Das Verhältnis von Mannose- zu Galactose-Monomeren unterscheidet sich je nach Art und Herkunft der Pflanze sowie nach der Temperatur, bei der sie gewachsen ist.

Im griechischen Bockshornklee (fenugreek gum) beträgt das Mannose-Galactose-Verhältnis in etwa 1:1 (entspricht einem Monomer Mannose zu einem Monomer Galactose); im Guar Gum in etwa 2:1; im Tara Gum in etwa 3:1; im Johannisbrotkernmehl (locust bean gum) in etwa 4:1 und im Cassia Gum in etwa 5:1.

Alle Galactomannane aus diesen Quellen sind für die kationische Modifizierung und Verwendung als Polymer c) in den erfindungsgemäßen kosmetischen Reinigungsmitteln geeignet.

Besonders geeignet für die Verwendung in den erfindungsgemäßen kosmetischen Mitteln sind Guar Gum und/oder Cassia Gum.

Wie die kationischen Inulin-Polymere b) können die Galactomannane, vorzugsweise Galactomannane aus den zuvor genannten Quellen, kationisch modifiziert werden, indem die Hydroxylgruppen der Galactomannanpolymere mit reaktiven quaternären Ammoniumverbindungen zur Reaktion gebracht werden. Als geeignete quaternäre Ammoniumverbindungen kommen vorzugsweise Verbindungen der nachfolgenden Formel

N⁺(R¹R²R³R⁴) X⁻

in Frage, in der
R¹, R² und R³ für Methyl- oder Ethylgruppen und
R⁴ für eine Epoxy-R⁵- oder eine Halohydringruppe Y-CH₂-CH(OH)-R⁵- steht, in der R⁵ eine C₁-C₃-Alkylengruppe, Y ein Halogenid und X ein Anion wie Cl⁻, Br, I⁻ oder HSO4⁻ ist.
Ganz besonders geeignete kationische Galactomannane-Polymere c) im Sinne der vorliegenden Erfindung entsprechen der Formel

   R-O-CH₂-CH(OH)-R⁵-N⁺(R¹R²R³) X⁻,
worin R das jeweilige Galactomannan und die anderen Reste dieselbe Bedeutung wie oben haben.

In einer besonders bevorzugten Ausführungsform enthalten die erfindungsgemäßen Haarreinigungsmittel daher kationische Galactomannan-Polymere c), die mit kationischen Hydroxy-C₁-C₃-alkyl-trialkylammonium-Gruppen, insbesondere mit Hydroxypropyltrimethylammoniumgruppen, kationisch modifiziert wurden.

Innerhalb dieser Ausführungsform sind Galactomannan-Polymere c) ganz besonders bevorzugt, die mit kationischen Hydroxy-C₁-C₃-alkyl-trialkylammonium-Gruppen, insbesondere mit Hydroxypropyltrimethylammoniumgruppen, kationisch modifiziert wurden und aus Guar Gum und/oder aus Cassia Gum stammen.

In einer ganz besonders bevorzugten Ausführungsform enthalten die erfindungsgemäßen kosmetischen Reinigungsmittel als kationisches Polymer c) mindestens eine der unter den INCI-Bezeichnungen Guar Hydroxypropyltrimonium Chloride, Hydroxypropyl Guar Hydroxypropyltrimonium Chloride und/oder Cassia Hydroxypropyltrimonium Chloride bekannten Verbindungen.

Unter diesen INCI-Bezeichnungen bekannte Guar- und Cassia-Polymere sind im Handel von verschiedenen Anbietern erhältlich, beispielsweise unter den Bezeichnungen Jaguar^{®}, N-Hance^{®}, Polycare^{®}, Clearhance^{®}, Activsoft^{®}, Guarquat^{®}, Vida-Care^{®}.

Jaguar^{®} C-162, Jaguar^{®} C500, Jaguar^{®} Styl 100, N-Hance^{®} 3196, N-Hance^{®} HPCG 1000, Activsoft^{®} C17, Guarquat^{®} C130 KC, Guarquat^{®} CP500 KC, Vida-Care^{®} GHTC und/oder Polycare^{®} Split Therapy sind spezifische Beispiele für erfindungsgemäß ganz besonders geeignete kationische Polymere natürlichen Ursprungs c).

Erfindungsgemäß geeignete kationische Polymere c) werden in den erfindungsgemäßen kosmetischen Reinigungsmitteln (bezogen auf deren Gesamtgewicht) bevorzugt in Mengen von 0,01 bis 2,00 Gew.-%, mehr bevorzugt 0,02 bis 0,90 Gew.-%, besonders bevorzugt 0,03 bis 0,80 Gew.-%, ganz besonders bevorzugt 0,04 bis 0,70 Gew.-% und insbesondere 0,05 bis 0,60 Gew.-% eingesetzt.

Besonders bevorzugt werden kationische Galactomannanpolymere c), ganz besonders bevorzugt aus Guar Gum und/oder Cassia Gum stammende kationische Galactomannanpolymere c), und insbesondere unter den INCI-Bezeichnungen Guar Hydroxypropyltrimonium Chloride, Hydroxypropyl Guar Hydroxypropyltrimonium Chloride und/oder Cassia Hydroxypropyltrimonium Chloride bekannte Verbindungen in den zuvor genannten Mengen in den erfindungsgemäßen Mitteln eingesetzt.

In einer weiteren Ausführungsform sind erfindungsgemäße Reinigungsmittel bevorzugt, die neben den kationischen Polymeren b) und c) keine weiteren kationischen Polymere enthalten.

Zur weiteren Optimierung der Konsistenz und/oder des optischen Erscheinungsbildes der erfindungsgemäßen kosmetischen Reinigungsmittel kann es von Vorteil sein, wenn ihnen weitere Komponenten, insbesondere Wachse, Trübungsmittel und/oder Perlglanzmittel hinzugefügt werden.

Unter geeigneten Wachsen d) im Sinne der vorliegenden Erfindung sind vorzugsweise kristalline, Hydroxylgruppen-enthaltende Glyceride, wie beispielsweise Trihydroxystearin, hydrierte Öle und/oder Modifikationen davon zu verstehen. Die Wachse d) können natürlichen, mineralischen und/oder synthetischen Ursprungs sein.

Geeignete Wachse d) bilden in den erfindungsgemäßen Zusammensetzungen ein Netzwerk aus, das die Koaleszenz und/oder Phasentrennung nicht ineinander löslicher Bestandteile in der Zusammensetzung verhindert.

Beispiele für besonders geeignete kristalline, Hydroxylgruppen-enthaltende Strukturierungsmittel sind im Sinne der vorliegenden Erfindung vor allem hydrierte Wachse auf Pflanzenbasis wie hydriertes (gehärtetes) Jojobaöl und/oder hydriertes (gehärtetes) Rizinusöl. Ganz besonders bevorzugt sind die im Handel beispielsweise unter den Handelbezeichnungen Cutina^{®} HR, Thixin^{®} oder Castorwax^{®} von mehreren Anbietern erhältliche Verbindungen. Insbesondere bevorzugt ist die unter der INCI-Bezeichnung Hydrogenated Castor Oil bekannte Verbindung.

Das oder die Wachs(e) d) kann (können) in den erfindungsgemäßen Reinigungsmitteln (bezogen auf deren Gesamtgewicht) bevorzugt in einer Menge von 0,01 bis 2,00 Gew.-%, mehr bevorzugt von 0,02 bis 1,50 Gew.-%, noch weiter bevorzugt von 0,03 bis 1,00 Gew.-%, besonders bevorzugt von 0,04 bis 0,75 Gew.-% und insbesondere von 0,05 bis 0,50 Gew.-% eigesetzt werden.

In einer weiteren bevorzugten Ausführungsform enthalten die erfindungsgemäßen Reinigungsmittel mindestens ein natürliches, mineralisches und/oder synthetisches Wachs d) in einem Gewichtsanteil von 0,01 bis 2,00 Gew.-% am Gesamtgewicht des kosmetischen Reinigungsmittels. Innerhalb dieser Ausführungsform ist es besonders bevorzugt, wenn das Wachs d) ausgewählt ist aus unter der INCI-Bezeichnung Hydrogenated Castor Oil bekannten Verbindungen.

Unter geeigneten Perlglanzmitteln ei) im Sinne der vorliegenden Erfindung werden vorzugsweise Mono- und/oder Diester des Ethylenglycols, 1,2-Propandiols und/oder Glycerins mit C₈-C₂₄-Fettsäuren und/oder und Ester von Polyethylenglycolen mit C₈-C₂₄-Fettsäuren verstanden. Bevorzugt sind Mono- und/oder Diester von Ethylenglycol und/oder PEG mit C₁₆-C₂₂-Fettsäuren, ganz besonders bevorzugt Mono- und/oder Diester von Ethylenglycol und/oder PEG mit Palmitinsäure, Stearinsäure und/oder Ölsäure, besonders bevorzugt Stearinsäure.

Beispiele für besonders bevorzugte Perlglanzmittel im Sinne der vorliegenden Erfindung sind: Glycol Distearate, wie beispielsweise das Handelsprodukt Cutina^{®} AGS der Firma Cognis, Glycol Monostearate, wie beispielsweise das Handelsprodukt Cutina^{®} EGMS der Firma Cognis, PEG-3 Distearate, wie beispielsweise das Handelsprodukt Genapol^{®} TS der Firma Clariant, PEG-2 Distearate, wie beispielsweise das Handelsprodukt Kessco^{®} DEGMS der Firma Akzo Nobel, Propylen Glycol Stearate, wie beispielsweise das Handelsprodukt Tegin^{®} P der Firma Goldschmidt. Weiterhin geeignete Perlglanzmittel ei) im Sinne der vorliegenden Erfindung können ausgewählt sein aus organischen und/oder anorganischen Pigmenten wie Eisenoxid, Titanoxid, Antimonoxid, Magnesiumoxid, Chromoxid, Aluminiumoxid, Zinkoxid, Zinkperoxid, Calciumaluminat, Kieselsäure, Magnesiumsilicoaluminat, Talg, synthetischem Glimmer, kolloidalem Kaolin, Bentonit, Zinklaurat, Polyvinylchlorid, Perlmutt, Kohlenstoffschwarz, Lanolin, Silica und/oder Mica. Zur Erzielung von Perlglanzeffekten ist es von Vorteil, wenn die Pigmente als feinteilige, pulverförmige Feststoffe eingesetzt werden. In einer besonders bevorzugten Ausführungsform handelt es sich bei dem feinteiligen, pulverförmigen Feststoff um Metalloxide, insbesondere Titandioxid, oder einen mit einem Metalloxid beschichteten synthetischen oder natürlichen Glimmer, wobei das Metalloxid bevorzugt ausgewählt wird aus Eisenoxid oder Titanoxid oder aus deren Mischungen. Beispiele für bevorzugte Glimmer-Pigmente sind beispielsweise Colorona Pigmente der Merck KGaA.

Unter geeigneten Trübungsmitteln eii) sind bevorzugt Styrol/Acrylates-Copolymere zu verstehen, wie sie beispielsweise die unter den Handelsbezeichnungen "Acudyne^{®}" oder "Acusol^{®}" von der Firma Dow oder "Acronal^{®}" von der Firma BASF erhältlich sind.

Perlglanzmittel ei) und/oder Trübungsmittel eii) können in den erfindungsgemäßen Reinigungsmitteln vorzugsweise in einem Gewichtsanteil von 0,05 bis 1,00 Gew.-% am Gesamtgewicht der kosmetischen Reinigungsmittel eingesetzt werden.

In einer weiteren bevorzugten Ausführungsform enthalten die erfindungsgemäßen Reinigungsmittel mindestens ein Trübungsmittel eii) und/oder mindestens ein Perlglanzmittel ei).

Innerhalb dieser Ausführungsform ist es besonders bevorzugt, wenn die erfindungsgemäßen Reinigungsmittel Glycol Distearate in einem Gewichtsanteil von 0,05 bis 1,00 Gew.-% am Gesamtgewicht des kosmetischen Reinigungsmittels enthalten.

Zur weiteren Steigerung der Pflegeeigenschaften der erfindungsgemäßen Reinigungsmittel ist es weiterhin von Vorteil, wenn diese zusätzlich zu den Pflegestoffen b) und c) mindestens ein natürliches Öl enthalten.

Eine weitere bevorzugte Ausführungsform der Erfindung ist deshalb dadurch gekennzeichnet, dass die kosmetischen Reinigungsmittel zusätzlich mindestens ein natürliches Öl f) in einem Gewichtsanteil von 0,1 bis 10 Gew.-% am Gesamtgewicht des kosmetischen Reinigungsmittels enthalten.

Geeignete Öle f) sind beispielsweise Amaranthsamenöl, Aprikosenkernöl, Arganöl, Avocadoöl, Babassuöl, Baumwollsaatöl, Borretschsamenöl, Camelinaöl, Canolaöl, Cranberryöl, Distelöl, Erdnussöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Kokosnussöl, Kürbiskernöl, Leinöl, Macadamianussöl, Maiskeimöl, Malvenöl, Mandelöl, Mangokernöl, Marulaöl, Mohnöl, Nachtkerzenöl, Olivenöl, Palmöl, Palmkernöl, Pfirsichkernöl, Rambutanöl, Rapssamenöl, Reiskleieöl, Rizinusöl, Sacha Inchi Öl, Safloröl, Sanddornfruchtfleischöl, Sanddornkernöl, Sasanquaöl, Sesamöl, Sheabutter, Sojaöl, Sonnenblumenöl, Teebaumöl, Traubenkernöl, Tsubakiöl, Walnussöl, Weizenkeimöl, Wiesenschaumkrautöl und/oder Wildrosenöl. Bevorzugt sind Aprikosenkernöl, Arganöl, Avocadoöl, Jojobaöl, Mandelöl, Rapsöl und/oder Sonnenblumenöl. Insbesondere bevorzugt ist Arganöl.

Das oder die pflanzliche(n) Öl(e) f) kann (können) in den erfindungsgemäßen kosmetischen Reinigungsmitteln (bezogen auf das Gewicht des gesamten Mittels) bevorzugt in einer Menge von 0,005 bis 1,00 Gew.-%, mehr bevorzugt von 0,01 bis 0,90 Gew.-%, besonders bevorzugt von 0,015 bis 0,80 Gew.-%, ganz besonders bevorzugt von 0,02 bis 0,70 Gew.-% und insbesondere von 0,025 bis 0,60 Gew.-% eingesetzt werden.

Für eine sehr gute (Kopf)hautverträglichkeit der erfindungsgemäßen Reinigungsmittel ist es von Vorteil, wenn diese einen leicht aciden pH-Wert aufweisen. Es wurde gefunden, dass erfindungsgemäße kosmetischen Reinigungsmittel in einem pH-Bereich von 4,0 bis 6,0 besonders gute Hautverträglichkeit und Milde aufweisen.

In einer weiteren bevorzugten Ausführungsform weisen die erfindungsgemäßen kosmetischen Reinigungsmittel daher einen pH-Wert im Bereich von 4,0 bis 6,0, mehr bevorzugt von 4,2 bis 5,9 und insbesondere bevorzugt von 4,5 - 5,8 auf.

Die erfindungsgemäßen kosmetischen Reinigungsmittel weisen gegenüber herkömmlichen (auf Silikonen basierenden) Formulierungen eine mindestens vergleichbare Produktperformance mit dem zusätzlichen Vorteil auf, dass keine weiteren Stabilisierungsmittel in die Reinigungsmittel eingearbeitet werden müssen, und dass auch bei regelmäßiger Anwendung der Mittel keine Überkonditionierung auftritt.

Erfindungsgemäße
kosmetische Reinigungsmittel sind deshalb im Wesentlichen frei von Silikonen und von weiteren Stabilisierungsmitteln, wie beispielsweise alkoxylierten nichtionischen Emulgatoren. Unter "im Wesentlichen frei" wird erfindungsgemäß verstanden, dass die erfindungsgemäßen kosmetischen Reinigungsmittel weniger als 0,25 Gew.-%, bevorzugt weniger als 0,10 Gew.-% und insbesondere gar keine Silikone und weitere Stabilisierungsmittel, wie beispielsweise alkoxylierte nichtionische Emulgatoren, enthalten (bezogen auf das Gesamtgewicht der kosmetischen Reinigungsmittel).

Dabei gelten die zuvor genannten Mengenangaben sowohl für frei zugesetztes Silikon und/oder weitere Stabilisierungsmittel, wie beispielsweise alkoxylierte nichtionische Emulgatoren, als auch für Silikone und/oder alkoxylierte nichtionische Emulgatoren, die gegebenenfalls als Nebenprodukt in Handelsprodukten enthalten sein können.

Neben den zuvor genannten wesentlichen und fakultativen Bestandteilen können die erfindungsgemäßen kosmetischen Reinigungsmittel in einer weiteren bevorzugten Ausführungsform zur weiteren Steigerung der pflegenden Eigenschaften der Mittel mindestens einen weiteren Pflegewirkstoff enthalten, der ausgewählt sein kann aus der Gruppe der
- Proteinhydrolysate und/oder
- Vitamine.

Unter geeigneten Proteinhydrolysaten sind Produktgemische zu verstehen, die durch sauer, basisch oder enzymatisch katalysierten Abbau von Proteinen (Eiweißen) erhalten werden können. Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Der Gewichtsanteil des oder der Proteinhydrolysats(e) am Gesamtgewicht der kosmetischen Reinigungsmittel beträgt bevorzugt 0,01 bis 5 Gew.-%, mehr bevorzugt 0,025 bis 3 Gew.-% und insbesondere 0,05 bis 2 Gew.-%.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   > Vitamin B₁ (Thiamin)
   > Vitamin B₂ (Riboflavin)
   > Vitamin B₃. Unter dieser Bezeichnung werden häufig die Verbindungen Nicotinsäure und Nicotinsäureamid (Niacinamid) geführt.
   > Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol. Der Gewichtsanteil des oder der Vitamins(e), Vitaminderivats(e), und/oder der Vitaminvorstufe(n) am Gesamtgewicht der kosmetischen Reinigungsmittel beträgt bevorzugt 0,001 bis 2 Gew.-%, besonders bevorzugt 0,005 bis 1 Gew.-% und insbesondere 0,01 bis 0,5 Gew.-%.

Es wurde festgestellt, dass die erfindungsgemäßen Mittel auch für eine Anwendung als Antischuppenzubereitung geeignet sind.

Der Gewichtsanteil an Antischuppenmitteln am Gesamtgewicht der erfindungsgemäßen Mittel kann bevorzugt 0,01 bis 10 Gew.-%, mehr bevorzugt 0,025 bis 7,5 Gew.-%, besonders bevorzugt 0,05 bis 5 Gew.-% und insbesondere 0,075 bis 3 Gew.-% betragen.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Bevorzugt sind Climbazol, Zink Pyrithion und Piroctone Olamine. Insbesondere bevorzugt ist Zink Pyrithion.

Weitere Wirk-, Hilfs- und Zusatzstoffe, die in den erfindungsgemäßen kosmetischen Reinigungsmitteln bevorzugt enthalten sein können, sind beispielsweise:
- Pflanzenextrakte,
- Feuchthaltemittel,
- Parfums,
- UV-Filter,
- Strukturanten wie Maleinsäure,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Wirkstoffe wie Bisabolol und/oder Allantoin,
- Komplexbildner wie EDTA, NTA, β-Alanindiessigsäure und Phosphonsäuren,
- Ceramide (N-Acylsphingosin - Fettsäureamide des Sphingosins oder synthetische Analogen solcher Lipide (Pseudo-Ceramide),
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure,
- Zusätzliche Viskositätsregler wie Salze (NaCl).

Ein zweiter Gegenstand der Erfindung ist kosmetische Verwendung des erfindungsgemäßen kosmetischen Reinigungsmittels zur Verbesserung der Haarpflegeeigenschaften, insbesondere zur Verbesserung der Kämmbarkeit, des Haargriffs, der Entwirrbarkeit und/oder des Haarglanzes. Bezüglich weiterer bevorzugter Ausführungsformen der erfindungsgemäßen Verwendung gilt mutatis mutandis das zu den erfindungsgemäßen Mitteln Gesagte.

Die folgenden Beispiele sollen den Gegenstand der vorliegenden Erfindung weiterhin erläutern.

### Patentbeispiele:

a) Es wurden die folgenden erfindungsgemäßen Reinigungszusammensetzungen hergestellt (die Mengenangaben beziehen sich auf Gew.-%):

| | **1** | **2** | **3** | **4** | **5** |
|---|---|---|---|---|---|
| a): Tensid* | 2,50 - 30,0 | 4,00 - 25,0 | 5,00 - 20,0 | 6,00 - 17,5 | 7,00 - 15,0 |
| b): kationisches Inulin-Polymer | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | | | | | |
|---|---|---|---|---|---|
| * anioniches, amphoteres, zwitterionisches und/oder nichtionisches Tensid | | | | | |

| | **6** | **7** | **8** | **9** | **10** |
|---|---|---|---|---|---|
| anioniches Tensid ai) | 2,50 - 30,0 | 4,00 - 25,0 | 5,00 - 20,0 | 6,00 - 17,5 | 7,00 - 15,0 |
| b): kationisches Inulin-Polymer | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **11** | **12** | **13** | **14** | **15** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| b): kationisches Inulin-Polymer | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **16** | **17** | **18** | **19** | **20** |
|---|---|---|---|---|---|
| Tensid* | 2,50 - 30,0 | 4,00 - 25,0 | 5,00 - 20,0 | 6,00 - 17,5 | 7,00 - 15,0 |
| Hydroxypropyltrimonium Inulin | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | | | | | |
|---|---|---|---|---|---|
| * anioniches, amphoteres, zwitterionisches und/oder nichtionisches Tensid | | | | | |

| | **21** | **22** | **23** | **24** | **25** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| Hydroxypropyltrimonium Inulin | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **26** | **27** | **28** | **29** | **30** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Hydroxypropyltrimonium Inulin | 0,01 - 5,00 | 0,02 - 4,00 | 0,03 - 3,00 | 0,04 - 2,50 | 0,05 - 2,00 |
| c): von b) verschiedenes kationisches Polymer | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **31** | **32** | **33** | **34** | **35** |
|---|---|---|---|---|---|
| Tensid* | 2,50 - 30,0 | 4,00 - 25,0 | 5,00 - 20,0 | 6,00 - 17,5 | 7,00 - 15,0 |
| b): kationisches Inulin-Polymer | 0,01 - 5,00 | 0,02 - 4,00 | 0,03 - 3,00 | 0,04 - 2,50 | 0,05 - 2,00 |
| Guar Hydroxypropyltrimonium Chloride, Hydroxypropyl Guar Hydroxypropyltrimonium Chloride und/oder Cassia Hydroxypropyltrimonium Chloride | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | | | | | |
|---|---|---|---|---|---|
| * anioniches, amphoteres, zwitterionisches und/oder nichtionisches Tensid | | | | | |

| | **36** | **37** | **38** | **39** | **40** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| Hydroxypropyltrimonium Inulin | 0,01 - 5,00 | 0,02 - 4,00 | 0,03 - 3,00 | 0,04 - 2,50 | 0,05 - 2,00 |
| Guar Hydroxypropyltrimonium Chloride, Hydroxypropyl Guar Hydroxypropyltrimonium Chloride und/oder Cassia Hydroxypropyltrimonium Chloride | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **41** | **42** | **43** | **44** | **45** |
|---|---|---|---|---|---|
| anioniches Tensid ai) | 2,50 - 20,0 | 3,00 - 18,0 | 4,00 - 16,0 | 5,00 - 14,0 | 6,00 - 12,5 |
| amphoteres und/oder zwitterionisches Tensid aii) | 1,00 - 10,00 | 1,20 - 9,00 | 1,40 - 8,00 | 1,60 - 7,00 | 1,80 - 6,00 |
| Hydroxypropyltrimonium Inulin | 0,01 - 5,00 | 0,02 - 4,00 | 0,03 - 3,00 | 0,04 - 2,50 | 0,05 - 2,00 |
| Guar Hydroxypropyltrimonium Chloride, Hydroxypropyl Guar Hydroxypropyltrimonium Chloride und/oder Cassia Hydroxypropyltrimonium Chloride | 0,01 - 2,00 | 0,02 - 0,90 | 0,03 - 0,80 | 0,04 - 0,70 | 0,05 - 0,60 |
| Wasser und ggfs. weitere Hilfs- und Zusatzstoffe | ad 100 | ad 100 | ad 100 | ad 100 | ad 100 |

| | **46** | **47** | **48** | **49** | **50** |
|---|---|---|---|---|---|
| Natriumlaurylethersulfat | 2,50 - 20,0 | 3,00 - 18,0 | 4,00 - 16,0 | 5,00 - 14,0 | 6,00 - 12,5 |
| Cocamidopropylbetain und/oder Disodium Cocoamphodiacetate | 1,00 - 10,00 | 1,20 - 9,00 | 1,40 - 8,00 | 1,60 - 7,00 | 1,80 - 6,00 |
| Hydroxypropyltrimonium Inulin | 0,01 - 5,00 | 0,02 - 4,00 | 0,03 - 3,00 | 0,04 - 2,50 | 0,05 - 2,00 |
| Guar Hydroxypropyltrimonium Chloride | 0,01 - 2,00 | | | 0,01 - 2,00 | |
| Hydroxypropyl Guar Hydroxypropyltrimonium Chloride | | 0,01 - 2,00 | | | |
| Cassia Hydroxypropyltrimonium Chloride | | | 0,01 - 2,00 | | 0,01 - 2,00 |
| Hydrogenated Castor Oil | 0,01 - 2,00 | 0,02 - 1,50 | 0,03 - 1,00 | 0,04 - 0,75 | 0,05 - 0,50 |
| Arganöl | 0,005 - 1,00 | 0,005 - 1,00 | | | 0,005 - 1,00 |
| Wasser und ggfs. weitere Hilfs- und Zusatzstoffe | ad 100 | ad 100 | ad 100 | ad 100 | ad 100 |

b) Beurteilung der von Haaren, die mit einem erfindungsgemäßen (I) und zwei nicht erfindungsgemäßen Haarreinigungsmitteln (II) und (III) gewaschen wurden

| **Inhaltsstoffe** | **I** | **II** | **III** |
|---|---|---|---|
| Sodium Laureth Sulfate | 10 | 10 | 10 |
| Disodium Cocoamphodiacetate | 0,6 | 0,6 | 0,6 |
| Cocamidoprolyl Betaine | 1,8 | 1,8 | 1,8 |
| Cocamide MEA | 0,4 | 0,4 | 0,4 |
| Hydrogenated Castor Oil | 0,1 | 0,1 | 0,1 |
| Guar Hydroxypropyltrimonium Chloride | 0,2 | | 0,2 |
| Hydroxypropyltrimonium Inulin | 0,3 | 0,3 | |
| Dimethicone | | | 0,4 |
| Jojobaöl | 0,1 | | |
| Parfum | 0,3 | 0,3 | 0,3 |
| NaCl | 1,0 | 1 | 1 |
| pH-Stellmittel, Konservierungsmittel | q.s. | q.s. | q.s. |
| Wasser | ad 100 | ad 100 | ad 100 |
| pH | 4,0-5,0 | 4,0-5,0 | 4,0-5,0 |

Die Shampoos I bis III wurden von erfahrenen Friseuren jeweils in einem Halbseitentest beurteilt (I gegen II und I gegen III). Jeweils 5,5 g der Shampoos I bis III wurden auf je eine Seite der nassen Haare aufgetragen, in üblicher Weise einmassiert und auf dieselbe Art und Weise mit denselben Mengen Wasser ausgespült. Anschließend beurteilten die Friseure verschiedene Haarpflegeparameter unter Vergabe der Noten 1 (schlecht) bis 6 (sehr gut). Die Ergebnisse sind der nachfolgenden Tabelle zu entnehmen, wobei aus den Einzelergebnissen der Bewertungen ein arithmetisches Mittel (Wilcoxon signed-rank test) gebildet wurde:

| **Haarpflegeparameter** | **I** | **II** | **III** | **Differenz I/II** | **Differenz I/III** |
|---|---|---|---|---|---|
| Haargriff während des Ausspülens | 4,00 | 3,75 | 3,67 | -0,25 | -0,33 |
| Entwirrbarkeit (nasse Haare) | 3,80 | 3,75 | 3,33 | -0,05 | -0,47 |
| Kämmbarkeit (nasse Haare) | 4,60 | 4,25 | 4,33 | -0,35 | -0,27 |
| Haargriff (nasse Haare) | 4,60 | 4,00 | 4,00 | -0,60 | -0,60 |
| Kämmbarkeit (trockene Haare) | 4,40 | 4,00 | 4,33 | -0,40 | -0,07 |
| Haargriff (trockene Haare) | 4,80 | 2,50 | 4,33 | -2,30 | -0,47 |
| Kontrolle der Elektrostatik | 4,20 | 3,75 | 3,67 | -0,45 | -0,53 |
| Glanz | 4,40 | 4,25 | 4,33 | -0,15 | -0,07 |
| Volumen | 4,40 | 4,50 | 4,33 | -0,10 | -0,07 |
| Gesamtbewertung | 4,35 | 3,86 | 4,03 | -0,49 | -0,32 |

## Patentansprüche

1. Kosmetisches Reinigungsmittel, enthaltend
a) mindestens ein Tensid, ausgewählt aus anionischen, amphoteren, zwitterionischen und/oder nichtionischen Tensiden,
b) mindestens ein kationisches Inulin-Polymer und
c) mindestens ein weiteres, von b) verschiedenes kationisches Polymer, ausgewählt aus kationischen Polygalactomannanderivaten, **dadurch gekennzeichnet, dass**
das kosmetische Reinigungsmittel (bezogen auf sein Gesamtgewicht) weniger als 0,25 Gew.-% Silikone und weniger als 0,25 Gew.-% alkoxylierte nichtionische Emulgatoren enthält.

2. Kosmetisches Reinigungsmittel nach Anspruch 1, enthaltend (bezogen auf das Gewicht des gesamten Mittels)
- 2,50 bis 30,00 Gew.-% mindestens eines Tensids a),
- 0,01 bis 5,00 Gew.-% mindestens eines kationischen Inulin-Polymeren b),
- 0,01 bis 2,00 Gew.-% mindestens eines von b) verschiedenen kationischen Polymeren c), ausgewählt aus kationischen Polygalactomannanderivaten.

3. Kosmetisches Reinigungsmittel nach einem der vorhergehenden Ansprüche, enthaltend (bezogen auf das Gewicht des gesamten Mittels)
ai) 2,5 bis 20 Gew.-% mindestens eines anionischen Tensids, und/oder
aii) 0,5 bis 10 Gew.-% mindestens eines amphoteren und/oder zwitterionischen Tensids.

4. Kosmetisches Reinigungsmittel nach Anspruch 3, wobei
ai) das anionische Tensid ausgewählt ist aus Alkyl(ether)sulfaten, Sarcosinaten, Tauraten, Isethionaten, Sulfosuccinaten und/oder Olefinsulfonaten, und
aii) das amphotere und/oder zwitterionische Tensid ausgewählt ist aus Alkylampho(di)acetaten und/oder -propionaten und/oder Alkylamidopropylbetainen.

5. Kosmetisches Reinigungsmittel nach einem der vorhergehenden Ansprüche, wobei das kationische Inulin-Polymer b) eine kationische Ladungsdichte > 1,5 meq/g, vorzugsweise > 2,0 meq/g, mehr bevorzugt > 2,5 meq/g, besonders bevorzugt > 3,0 meq/g und insbesondere > 3,5 meq/g aufweist.

6. Kosmetisches Reinigungsmittel nach einem der vorhergehenden Ansprüche, wobei das kationische Inulin-Polymer b) eine mittlere Molmasse von 2,000 bis 50,000 g/mol, vorzugsweise von 2,500 bis 40,000 g/mol, mehr bevorzugt 3,000 bis 30,000 g/mol, besonders bevorzugt 3,500 bis 20,000 g/mol und insbesondere 4,000 bis 10,000 g/mol aufweist.

7. Kosmetisches Reinigungsmittel nach einem der vorhergehenden Ansprüche, wobei das kationische Inulin-Polymer b) mit Hydroxy-C₁-C₃-alkyl-trialkylammonium-Gruppen kationisch modifiziert ist, vorzugsweise wobei das kationische Inulin-Polymer b) ausgewählt ist aus unter der INCI-Bezeichnung Hydroxypropyltrimonium Inulin bekannten Verbindungen.

8. Kosmetisches Reinigungsmittel nach einem der vorhergehenden Ansprüche, wobei das kationische Polymer c) ausgewählt ist aus unter der INCI-Bezeichnung Guar Hydroxypropyltrimonium Chloride und/oder Hydroxypropyl Guar Hydroxypropyltrimonium Chloride und/oder Cassia Hydroxypropyltrimonium Chloride bekannten Verbindungen.

9. Kosmetisches Reinigungsmittel nach einem der vorhergehenden Ansprüche, enthaltend mindestens ein natürliches, mineralisches und/oder synthetisches Wachs d) in einem Gewichtsanteil von 0,01 bis 2,00 Gew.-% am Gesamtgewicht des kosmetischen Reinigungsmittels, vorzugsweise ein unter der INCI-Bezeichnung Hydrogenated Castor Oil bekanntes Wachs d).

10. Kosmetisches Reinigungsmittel nach einem der vorhergehenden Ansprüche, enthaltend mindestens ein Trübungsmittel ei) und/oder mindestens ein Perlglanzmittel eii).

11. Kosmetisches Reinigungsmittel nach Anspruch 10, enthaltend Glycol Distearate in einem Gewichtsanteil von 0,05 bis 1,00 Gew.-% am Gesamtgewicht des kosmetischen Reinigungsmittels.

12. Kosmetische Verwendung eines kosmetischen Reinigungsmittels nach einem der Ansprüche 1 bis 11 zur Verbesserung der Haarpflegeeigenschaften, insbesondere zur Verbesserung der/des
a. Kämmbarkeit,
b. Haargriffs,
c. Entwirrbarkeit,
d. Haarglanzes.

## Claims

1. A cosmetic cleansing agent, containing
a) at least one surfactant selected from anionic, amphoteric, zwitterionic and/or non-ionic surfactants,
b) at least one cationic inulin polymer, and
c) at least one additional cationic polymer, different from b), selected from cationic polygalactomannan derivatives, **characterized in that**
the cosmetic cleaning agent contains, based on its total weight, less than 0.25 wt.% of silicones and less than 0.25 wt.% of alkoxylated non-ionic emulsifiers.

2. The cosmetic cleaning agent according to claim 1, containing, based on the weight of the total agent,
- 2.50 to 30.00 wt.% of at least one surfactant a),
- 0.01 to 5.00 wt.% of at least one cationic inulin polymer b),
- 0.01 to 2.00 wt.% of at least one cationic polymer c), different from b), selected from cationic polygalactomannan derivatives.

3. The cosmetic cleaning agent according to one of the preceding claims, containing, based on the weight of the total agent,
ai) 2.5 to 20 wt.% of at least one anionic surfactant, and/or
aii) 0.5 to 10 wt.% of at least one amphoteric and/or zwitterionic surfactant.

4. The cosmetic cleansing agent according to claim 3, wherein
ai) the anionic surfactant is selected from alkyl (ether) sulfates, sarcosinates, taurates, isethionates, sulfosuccinates and/or olefin sulfonates, and
aii) the amphoteric and/or zwitterionic surfactant is selected from alkyl ampho(di)acetates and/or -propionates, and/or alkylamidopropyl betaines.

5. The cosmetic cleaning agent according to one of the preceding claims, wherein the cationic inulin polymer b) has a cationic charge density > 1.5 meq/g, preferably > 2.0 meq/g, more preferably > 2.5 meq/g, particularly preferably > 3.0 meq/g and in particular > 3.5 meq/g.

6. The cosmetic cleaning agent according to one of the preceding claims, wherein the cationic inulin polymer b) has an average molar mass of 2.000 to 50.000 g/mol, preferably of 2.500 to 40.000 g/mol, more preferably 3.000 to 30.000 g/mol, particularly preferably 3.500 to 20.000 g/mol and in particular 4.000 to 10.000 g/mol.

7. The cosmetic cleaning agent according to one of the preceding claims,
wherein the cationic inulin polymer b) is cationically modified with hydroxy-C₁-C₃-alkyl-trialkylammonium groups, preferably wherein the cationic inulin polymer b) is selected from compounds known under the INCI name Hydroxypropyltrimonium Inulin.

8. The cosmetic cleaning agent according to one of the preceding claims, wherein the cationic polymer c) is selected from compounds known under the INCI name Guar Hydroxypropyltrimonium Chloride and/or Hydroxypropyl Guar Hydroxypropyltrimonium Chloride and/or Cassia Hydroxypropyltrimonium Chloride.

9. The cosmetic cleaning agent according to one of the preceding claims, containing at least one natural, mineral and/or synthetic wax d) in a weight proportion of 0.01 to 2.00 wt.% of the total weight of the cosmetic cleaning agent, preferably a wax d) known under the INCI name Hydrogenated Castor Oil.

10. The cosmetic cleaning agent according to one of the preceding claims, containing at least one opacifying agent ei) and/or at least one pearlescing agent eii).

11. The cosmetic cleaning agent according to claim 10, containing glycol distearate in a weight proportion of 0.05 to 1.00 wt.% of the total weight of the cosmetic cleaning agent.

12. A cosmetic use of a cosmetic cleaning agent according to one of claims 1 to 11 for improving hair care properties, in particular for improving the
a. combability,
b. hair feel,
c. detangling,
d. hair shine.

## Revendications

1. Agent de nettoyage cosmétique, contenant
a) au moins un tensioactif choisi parmi des tensioactifs anioniques, amphotères, zwitterioniques et/ou non ioniques,
b) au moins un polymère d'inuline cationique et
c) au moins un autre polymère cationique différent de b), choisi parmi des dérivés de polygalactomannane cationiques, **caractérisé en ce que**
l'agent de nettoyage cosmétique contient (par rapport à son poids total) moins de 0,25 % en poids de silicones et moins de 0,25 % en poids d'émulsifiants non ioniques alcoxylés.

2. Agent de nettoyage cosmétique selon la revendication 1, contenant (par rapport au poids de l'ensemble de l'agent)
- 2,50 à 30,00 % en poids d'au moins un tensioactif a),
- 0,01 à 5,00 % en poids d'au moins un polymère d'inuline cationique b),
- 0,01 à 2,00 % en poids d'au moins un polymère cationique c) différent de b), choisi parmi des dérivés de polygalactomannane cationiques.

3. Agent de nettoyage cosmétique selon l'une des revendications précédentes, contenant (par rapport au poids de l'ensemble de l'agent)
ai) 2,5 à 20 % en poids d'au moins un tensioactif anionique, et/ou
aii) 0,5 à 10 % en poids d'au moins un tensioactif amphotère et/ou zwitterionique.

4. Agent de nettoyage cosmétique selon la revendication 3, dans lequel
ai) le tensioactif anionique est choisi parmi (éther)sulfates d'alkyle, sarcosinates, taurates, iséthionates, sulfosuccinates et/ou sulfonates d'oléfine, et
aii) le tensioactif amphotère et/ou zwitterionique est choisi parmi alkylampho(di)acétates et/ou alkylampho(di)propionates et/ou alkylamidopropylbétaïnes.

5. Agent de nettoyage cosmétique selon l'une des revendications précédentes, dans lequel le polymère d'inuline cationique b) présente une densité de charge cationique > 1,5 meq/g, de préférence > 2,0 meq/g, plus préférentiellement > 2,5 meq/g, de manière particulièrement préférée > 3,0 meq/g et en particulier > 3,5 meq/g.

6. Agent de nettoyage cosmétique selon l'une des revendications précédentes, dans lequel le polymère d'inuline cationique b) présente une masse molaire moyenne allant de 2,000 à 50,000 g/mol, de préférence de 2,500 à 40,000 g/mol, plus préférentiellement de 3,000 à 30,000 g/mol, de manière particulièrement préférée de 3,500 à 20,000 g/mol et en particulier de 4,000 à 10,000 g/mol.

7. Agent de nettoyage cosmétique selon l'une des revendications précédentes, dans lequel le polymère d'inuline cationique b) est modifié de manière cationique avec des groupes hydroxy-alkyl-C₁-C₃-trialkylammonium, de préférence dans lequel le polymère d'inuline cationique b) est choisi parmi les composés connus sous la dénomination INCI Hydroxypropyltrimonium Inulin.

8. Agent de nettoyage cosmétique selon l'une des revendications précédentes, dans lequel le polymère cationique c) est choisi parmi les composés connus sous la dénomination INCI Guar Hydroxypropyltrimonium Chloride et/ou Hydroxypropyl Guar Hydroxypropyltrimonium Chloride et/ou Cassia Hydroxypropyltrimonium Chloride.

9. Agent de nettoyage cosmétique selon l'une des revendications précédentes, contenant au moins une cire d) naturelle, minérale et/ou synthétique dans une proportion en poids allant de 0,01 à 2,00 % en poids par rapport au poids total de l'agent de nettoyage cosmétique, de préférence une cire d) connue sous la dénomination INCI Hydrogenated Castor Oil.

10. Agent de nettoyage cosmétique selon l'une des revendications précédentes, contenant au moins un agent opacifiant ei) et/ou au moins un agent nacrant eii).

11. Agent de nettoyage cosmétique selon la revendication 10, contenant du distéarate de glycol en une proportion en poids allant de 0,05 à 1,00 % en poids par rapport au poids total de l'agent de nettoyage cosmétique.

12. Utilisation cosmétique d'un agent de nettoyage cosmétique selon l'une des revendications 1 à 11 pour l'amélioration des propriétés de soin des cheveux, en particulier pour l'amélioration
a. du fait de pouvoir peigner,
b. de la densité de cheveux,
c. du démêlage,
d. de l'éclat des cheveux.
